Europäisches Patentamt

(19) European Patent Office

Office européen des brevets

(11) Publication number: **0 022 922**
**B1**

## (12) EUROPEAN PATENT SPECIFICATION

(45) Date of publication of patent specification: **11.05.83**

(51) Int. Cl.³: **A 61 M 1/03**

(21) Application number: **80103136.0**

(22) Date of filing: **06.06.80**

(54) Method of forming an aqueous dialysis solution.

(30) Priority: **24.07.79 DE 2929871**

(43) Date of publication of application:
**28.01.81 Bulletin 81/4**

(45) Publication of the grant of the patent:
**11.05.83 Bulletin 83/19**

(84) Designated Contracting States:
**AT BE CH DE FR GB IT LI LU NL SE**

(56) References cited:
FR - A - 1 546 072
FR - A - 2 237 639
FR - A - 2 271 864
GB - A - 1 368 566
US - A - 3 525 686
US - A - 3 560 380
US - A - 4 085 046
US - A - 4 136 708

(73) Proprietor: **Gambro Dialysatoren K.G.**
**Ermelesstrasse 76**
**D-7450 Hechingen (DE)**

(72) Inventor: **Gullberg, Claes-Ake**
**Fürstenstrasse 1**
**D-7450 Hechingen (DE)**
Inventor: **von Dobrogoiski, Alexander**
**Lehmgrubenstrasse 35**
**D-7451 Rangendingen (DE)**

(74) Representative: **Weber, Dieter, Dr. et al,**
**Dr. Dieter Weber und Klaus Seiffert**
**Patentanwälte Gustav-Freytag-Strasse 25**
**D-6200 Wiesbaden 1 (DE)**

Courier Press, Leamington Spa, England.

It is known to use haemo dialysis solutions which contain sodium bicarbonate together with sodium chloride and optionally other alkali and alkali earth chlorides. But there has been found as disadvantage of such dialysis solutions containing bicarbonate that calcium and magnesium carbonates may precipitate. In order to avoid these disadvantages, different methods have been used.

One method was to replace sodium acetate for the bicarbonate since the alkali earth acetates are easily soluble, so that no precipitates of slightly soluble calcium or magnesium compounds result. But since the acetate is partly transferred into the blood, sometimes the patients show acidosis which makes undesirable the use of acetate instead of bicarbonate in hemodialysis solutions. Another method was that, to bubble carbon dioxide into the dialysis solution in order to maintain the pH value of the dialysis solution in the acidic range and to suppress the formation of slightly soluble magnesium and calcium carbonates. But bubbling carbon dioxide into a dialysis solution is a process complicated and capable of interferences, and in this method the introduced amount of carbon dioxide and thus the pH value can be controlled only relatively bad.

Additionally the physicians prefer storable concentrates for dialysis solutions which concentrates have only to be diluted by water to become useful. But concentrates of aqueous sodium bicarbonate solutions containing calcium ions are not stable and only slightly bacteriostatic.

Further the US—A—35 60 380 discloses dry concentrates for hemodialysis solutions having a total moisture content not substantially more than about 0,75% by weight. But when such dry concentrates are dissolved in water, smaller portions of some of the components may remain undissolved, and dissolving is time consuming.

The object of the invention was to obtain dialysis solutions which may be left standing for some time without the necessity of bubbling carbon dioxide into the solution, by means of storable and bacteriostatic concentrate solutions. Moreover the dialysis solutions shall contain at most small amounts of acetate.

The inventive method of forming an aqueous dialysis solution containing sodium chloride, carbonate or hydrocarbonate anions, respectively, calcium cations and optionally magnesium and/or potassium cations is characterized by mixing an aqueous solution (A) of sodium carbonate or bicarbonate with an aqueous solution (B) containing the calcium cations and, if magnesium cations are contained, the magnesium cations, as well as an acid which forms physiologically compatible sodium, calcium and optionally potassium and magnesium salts, and with dilution water.

According to a first embodiment of the invention the solution (A) contains sodium carbonate and the solution (B) contains hydrochloric acid as the acid.

It is unusual to use strong acids such as hydrochloric acid in physiological aqueous solutions. But the said acid is immediately reacted when combined with the aqueous solution containing the sodium carbonate, whereby sodium chloride is formed. During that reaction the sodium carbonate forms the equivalent amount of sodium bicarbonate, and in the event of a corresponding proportionation of the amount of hydrochlorid acid, a smaller part of the said sodium bicarbonate is decomposed to form additional sodium chloride and carbon dioxide which has the function of the carbon dioxide which is usually bubbled into the solution and adjusts the pH value of the dialysis solution.

Thus since the hydrochloric acid contained in the solution (B) serves on the one hand completely to convert the sodium carbonate to sodium bicarbonate and on the other hand to decompose a smaller portion of the formed sodium bicarbonate forming carbon dioxide, it is preferred that the solution (B) contains 1,05 to 1,3, preferably 1,05 to 1,2 mole of HCl per mole of $Na_2CO_3$ in the solution (A). One mole of the said hydrochloric acid is necessary to convert quantitatively the sodium carbonate to sodium bicarbonate, whereas the remaining amount of hydrochloric acid serves to release $CO_2$.

The amount of sodium chloride formed during the said reactions may be calculated, so that there is added to the solutions (A) and/or (B) the amount of sodium chloride necessary for the dialysis solution, less the amount of sodium chloride formed in the above reactions. The said sodium chloride which is necessary in the dialysis solution to make it substantially isotonic with the blood liquid, may be added to each of the both solutions (A) and (B) or partially to both. Additionally there are added calcium chloride and optionally magnesium chloride and potassium chloride to the concentrate in the usual manner. The amounts of these compounds in the dialysis solutions are known and are not distinguished from the prior art. However, it is suitable to have in the solution (A) only sodium carbonate, but in the solution (B) additionally to the hydrochloric acid all other compounds, since then the solution (A) may be used as standard stock solution for solutions (B) having different concentration, if only the concentration of hydrochloric acid thereof is in conformity with the concentration of sodium carbonate in the solution (A) corresponding to the above statements.

Since smaller amounts of acetate are not dangerous, it is also possible to replace acetic acid for a smaller portion of the hydrochloric acid.

For example per mole of sodium carbonate in the solution (A) 1 mole of HCl and 0,05 to 0,3 mole of acetic acid should be present in the solution (B).

According to a second embodiment of the invention the solution (A) contains sodium bicarbonate.

It is surprising that the sodium bicarbonate solutions designated as aqueous solution (A) are stable during long time storage and result in stable dialysis solutions without bubbling carbon dioxide into the solutions to stabilize them.

For this effect it is sufficient that only relatively small amounts of an acid which forms the stated physiologically compatible alkali and alkali earth salts are used. This results in the advantage for example in the event of the use of acetic acid, that the ready dialysis solution may contain only small amounts of alkali acetate which do not result in acidosis. Preferably the aqueous solution (B) contains 0,01 to 0,25, especially 0,03 to 0,15, such as for example about 0,05 mole of the stated acid per mole of sodium bicarbonate in the aqueous solution (A).

By the combination of the two solutions (A) and (B) with each other and with dilution water the hydrogen ions react with a small part of the sodium bicarbonate forming $CO_2$ which remains in the dialysis solution and stabilizes it by avoiding a formation of alkali earth carbonates and by adjusting the pH value of the dialysis solution.

An acid in the aqueous solution (B) inorganic or organic acids may be used, which form physiologically compatible sodium and calcium salts and, if the aqueous concentrates contain magnesium and potassium cations, form physiologically compatible magnesium and potassium salts, too. Such acids which may be used according to the invention are for example acetic acid, citric acid, lactic acid, hydrochloric acid and/or amino acids.

Acetic acid is preferred among the said acids, if sodium bicarbonate is used in the solution (A). The said acetic acid may be used without hesitation, since the amount thereof is relatively small in comparison with the amount of hydrocarbonate, so that from the dialysis solution only neglectible amounts of acetate can be transferred into the blood of the patient, so that no risk of acidosis exists.

The sodium chloride may be contained in both aqueous solutions (A) and/or (B), but it is preferably present in the solution (A) in a concentration as high as possible.

The sodium chloride increases the autosterility of the aqueous solutions. If the aqueous solution (A) contains a great portion of sodium cations, by different dosage of the relative amount of the solution (A) at the combination with the solution (B) and with dilution water, optional millival values for the sodium in the dialysis solution may be adjusted without having stock solutions of different concentrations. That by the superdosage or underdosage relating to the solution (A) also the other components such as sodium bicarbonate, are super or under dosed, is no disadvantage, since the bicarbonate is rapidly decomposed by the body.

It may be suitable, if the aqueous solution (B) contains additionally glucose. The amount of the glucose in the solution (B) is suitably 5 to 400 gs/l, preferably 15 to 80 gs/l.

An increased storage stability of the solution (A) is obtained, if it is maintained in such a container and under such conditions, that no or only small escape of $CO_2$ from the solution and the gaseous phase above the said solution out of the said container is possible. This may be reached in a different manner. For example the container can be manufactured of a material or with such a thickness of the wall that no $CO_2$ or only a very small amount of $CO_2$ escapes from the solution through the wall of the container. Another suitable method is to store the solution (A) in comparably big containers, so that the ratio of the volume to the surface area of the liquid body is relatively great. Thereby the interface between the solution and the wall of the container, where $CO_2$ may escape by diffusion, is relatively small in comparison with the volume of the solution. This is especially important, if for the storage of the solution plastic containers must be used for which a diffusion of $CO_2$ through the wall of the container cannot be excluded. It is surprising that by the choice of big storage containers the bicarbonate solution (A) remains completely stable for long periods of time. The stability of the said solution can still be improved by a storage at low temperatures.

The method according to the invention has several important advantages over known methods for obtaining dialysis solutions. A first advantage is that the solutions (A) and (B) are completely stable at the storage during a long period of time as well as the ready dialysis solutions have a longer storage stability without the precipitation of slightly soluble alkali earth compounds and without turbidity.

Another advantage is that by the solutions (A) and (B) according to the invention dialysis solutions are obtained which do not result in any physiological disadvantage, such as acidosis.

Finally a high autosterility and a good flexibility of the dosage are obtained, so that optional sodium concentrations in the dialysis solution may be adjusted without having stock solutions of different concentration.

In the practical use both aqueous solutions (A) and (B) are mixed together and with the dilution water separately from the dialysis device or within the dialysis device immediately before the membrane, whereby suitably the solution (A) is diluted with water and then the solution (B) is metered. This may be done suitably by forced dosage in order to adjust the correct ratio of both solutions (A) and (B), whereby in the mixing device the solution obtains the necessary temperature. Moreover as safety precaution a conductivity and optionally a pH value, density- and/or flow control device may be provided. If desired, the feed of the solutions (A) and/or (B) may be made dependent from the above mentioned measured values, and by these measurements the feed may be controlled.

Example 1
Solution (A):
    water 1 l
    NaHCO$_3$ 132,3 gs (1575 mmole)
    sodium chloride 61,36 gs (1050 mmole)
    filling up with water to a total volume of 2 l

Solution (B):
    water 0,5 l
    acetic acid 10,517 gs (175 mmole)
    NaCl 122,72 gs (2100 mmole)
    KCl 5,227 gs (70 mmole)
    MgCl$_2$ . 6H$_2$O 7,116 gs (35 mmole)
    CaCl$_2$ . 6H$_2$O 13,42 gs (16,25 mmole)
    filling up with water to a total volume of 1 l

During the combination of both aqueous solutions there has been diluted with 32 l of water to totally 35 l of dialysis solution. This solution contained 5 mmole CO$_2$, and in a surprising manner this amount was sufficient to maintain the dialysis solution stable during storage for a longer time, too.

Example 2
Solution (A):
    water 1000 l
    Na$_2$CO$_3$ water-free 2 11,47 kg
    filling up with water to a total volume of 1500 l

Solution (B):
    water 750 l
    NaCl 181,0 kg
    HCl, 37%ig, 212, 1 kg
    MgCl$_2$ . 6H$_2$O 10,674 kg
    CaCl$_2$ . 6H$_2$O 20,13 kg
    filling up with water to a total volume of 1500 l

**Claims**

1. A method of forming an aqueous dialysis solution containing sodium chloride, carbonate or hydrocarbonate anions respectively, calcium cations and optionally magnesium and/or potassium cations, characterized by mixing an aqueous solution (A) containing sodium carbonate or bicarbonate with an aqueous solution (B) containing the calcium cations and, if magnesium cations are contained, the magnesium cations, as well as an acid which forms physiologically compatible sodium, calcium and optionally potassium and magnesium salts, and with dilution water.

2. A method according to claim 1 characterized in that the aqueous solution (A) contains sodium carbonate and the aqueous solution (B) contains hydrochloric acid as acid.

3. A method according to claim 2, characterized in that the solution (B) contains 1,05 to 1,3, preferably 1,05 to 1,2 mole HCl per mole Na$_2$CO$_3$ in the solution (A).

4. A method according to claim 2 or 3, characterized in that the solution (A) and/or the solution (B) contain the sodium chloride and optionally the potassium chloride.

5. A method according to any of claims 2 through 4, characterized in that up to 0,3 mole-% of the HCl are replaced by acetic acid.

6. A method according to claim 1, characterized in that the aqueous solution (A) contains sodium bicarbonate.

7. A method according to claim 6, characterized in that the solution (B) contains 0,01 to 0,25, preferably 0,03 to 0,15 mole of the acid per mole of sodium bicarbonate in the solution (A).

8. A method according to any of claims 6 through 7, characterized in that the solution (B) contains as acid acetic acid, citric acid, lactic acid, hydrochloric acid and/or an amino acid.

9. A method according to any of claims 6 through 8, characterized in that the solution (B) contains additionally glucose, preferably 5 to 400 gs/l, especially 15 to 80 gs/l.

10. A method according to any of claims 1 through 9, characterized in that the solution (A) is contained in a container which enables no or only a little escape of CO$_2$ from the solution and the gaseous phase thereover.

**Patentansprüche**

1. Verfahren zur Herstellung einer Natriumchlorid, Carbonat bzw. Hydrogencarbonatanionen, Calciumkationen und gegebenenfalls Magnesium- und/oder Kaliumkationen enthaltenden wäßrigen Dialyselösung, dadurch gekennzeichnet, daß man eine Natriumcarbonat oder -bicarbonat enthaltende wäßrige Lösung (A) mit einer die Calciumkationen und, wenn Magnesiumkationen enthalten sind, die Magnesiumkationen sowie eine Säure, die physiologisch verträgliche Natrium-, Calcium- und gegebenenfalls Kalium- und Magnesiumsalze bildet, enthaltenden wäßrigen Lösung (B) und mit Verdünnungswasser vermischt.

2. Verfahren nach Anspruch 1, dadurch gekennzeichnet, daß die wäßrige Lösung (A) Natriumcarbonat und die wäßrige Loßung (B) als Säure Salzsäure enthält.

3. Verfahren nach Anspruch 2, dadurch gekennzeichnet, daß die Lösung (B) 1,05 bis 1,3, vorzugsweise 1,05 bis 1,2 Mol HCl je Mol Na$_2$CO$_3$ in der Lösung (A) enthält.

4. Verfahren nach Anspruch 2 oder 3, dadurch gekennzeichnet, daß die Lösung (A) und/oder die Lösung (B) das Natrium-chlorid und gegebenenfalls das Kaliumchlorid enthält.

5. Verfahren nach einem der Ansprüche 2 bis 4, dadurch gekennzeichnet, daß bis zu 0,3 Mol-% des HCl durch Essigsäure ersetzt sind.

6. Verfahren nach Anspruch 1, dadurch gekennzeichnet, daß die wäßrige Lösung (A) Natriumcarbonat enthält.

7. Verfahren nach Anspruch 6, dadurch gekennzeichnet, daß die Lösung (B) 0,01 bis 0,25, vorzugsweise 0,03 bis 0.15 Mol der Säure je Mol Natriumbicarbonat in der Lösung (A) enthält.

8. Verfahren nach einem der Ansprüche 6 und 7, dadurch gekennzeichnet, daß die Lösung (B) als Säure Essigsäure, Zitronensäure, Milchsäure, Salzsäure und/oder eine Aminosäure enthält.

9. Verfahren nach einem der Ansprüche 6 bis 8, dadurch gekennzeichnet, daß die Lösung (B) zusätzlich Glucose, vorzugsweise 5 bis 400 g/l, besonders 15 bis 80 g/l, enthält.

10. Verfahren nach einem der Ansprüche 1 bis 9, dadurch gekennzeichnet, daß die Lösung (A) in einem Behältnis enthalten ist, das kein oder nur geringes Entweichen von $CO_2$ aus der Lösung und der darüber befindlichen Gasphase ermöglicht.

**Revendications**

1. Procédé de préparation d'une solution aqueuse de dialyse contenant du chlorure de sodium, et respectivement des anions de carbonate ou de carbonate acide, et des cations de calcium, et, éventuellement de magnésium et/ou de potassium, caractérisé en ce qu'on mélange une solution aqueuse (A) de carbonate ou bicarbonate de sodium avec une solution aqueuse (B) contenant les cations calcium, et, dans le cas de leur présence, les cations magnésium, et avec un acide qui forme des sels physiologiquement compatibles de sodium, calcium et éventuellement de potassium et de magnésium, ainsi qu'avec l'eau de dilution.

2. Procédé selon la revendication 1, caractérisé en ce que la solution aqueuse (A) contient du carbonate de sodium et la solution aqueuse (B) contient, comme acide, de l'acide chlorhydrique.

3. Procédé selon la revendication 2, caractérisé en ce que la solution aqueuse (B) contient 1,05 à 1,3, de préférence 1,05 à 1,2 moles de HCl par mole de $Na_2CO_3$ dans la solution (A).

4. Procédé selon la revendication 2 ou 3, caractérisé en ce que la solution (A) et/ou la solution (B) contient le chlorure de sodium et éventuellement le chlorure de potassium.

5. Procédé selon l'une des revendications 2 à 4, caractérisé en ce qu'on remplace jusqu'à 0,3 moles de HCl par de l'acide acétique.

6. Procédé selon la revendication 1, caractérisé en ce que la solution aqueuse (A) contient du bicarbonate de sodium.

7. Procédé selon la revendication 6, caractérisé en ce que la solution (B) contient de 0,01 à 0,25, de préférence 0,03 à 0,15 moles de l'acide, par mole du bicarbonate de sodium dans la solution (A).

8. Procédé selon l'une des revendications 6 et 7, caractérisé en ce que la solution (B) contient, comme acide, de l'acide acétique, citrique, lactique, chlorhydrique et/ou un amino-acide.

9. Procédé selon l'une des revendications 6 à 8, caractérisé en ce que la solution (B) contient, en outre, du glucose, de préférence dans la proportion de 5 à 400 g/l, encore mieux de 15 à 80 g/l.

10. Procédé selon l'une des revendications 1 à 9, caractérisé en ce que la solution (A) est contenue dans un récipient tel que pas, ou très peu, de $CO_2$ puisse s'échapper de la solution et de la phase gazeuse qui la surmonte.